(19) **Europäisches Patentamt**
**European Patent Office**
**Office européen des brevets**

(11) **EP 3 874 262 B1**

(12) **EUROPEAN PATENT SPECIFICATION**

(45) Date of publication and mention
of the grant of the patent:
**19.02.2025 Bulletin 2025/08**

(21) Application number: **19879827.4**

(22) Date of filing: **29.10.2019**

(51) International Patent Classification (IPC):
***G01N 33/04*** *(2006.01)* ***C12Q 1/37*** *(2006.01)*

(52) Cooperative Patent Classification (CPC):
**C07K 14/4732; C12Q 1/37; G01N 30/06;**
**G01N 33/68;** G01N 30/72; G01N 2030/8813;
G01N 2333/4731; G01N 2333/976

(86) International application number:
**PCT/NZ2019/050143**

(87) International publication number:
**WO 2020/091608 (07.05.2020 Gazette 2020/19)**

(54) **BETA-CASEIN ANALYSIS OF MILK AND MILK PRODUCTS**

BETA-KASEIN-ANALYSE VON MILCH UND MILCHPRODUKTEN

ANALYSE DE BÊTA-CASÉINE DE LAIT ET DE PRODUITS LAITIERS

(84) Designated Contracting States:
**AL AT BE BG CH CY CZ DE DK EE ES FI FR GB**
**GR HR HU IE IS IT LI LT LU LV MC MK MT NL NO**
**PL PT RO RS SE SI SK SM TR**

(30) Priority: **29.10.2018 US 201862752080 P**

(43) Date of publication of application:
**08.09.2021 Bulletin 2021/36**

(73) Proprietor: **The A2 Milk Company Limited**
**Auckland 1010 (NZ)**

(72) Inventors:
• **COONEY, Terence Patrick**
**Cambridge 3432 (NZ)**
• **JAINE, Jacob Evan**
**Glenview, Hamilton 3206 (NZ)**

(74) Representative: **EIP**
**Fairfax House**
**15 Fulwood Place**
**London WC1V 6HU (GB)**

(56) References cited:
**CN-A- 108 519 485**

• **PRIYADARSHINI PRIYANKA ET AL: "Impact of**
**milk protein on human health: A1 verses A2",**
**INTERNATIONAL JOURNAL OF CHEMICAL**
**STUDIES, 1 January 2018 (2018-01-01),**
**XP055923823**
• **DUARTE-VAZQUEZ, MA. ET AL.: "Production of**
**Cow's Milk Free from Beta-Casein A1 and Its**
**Application in the Manufacturing of Specialized**
**Foods for Early Infant Nutrition", FOODS, vol. 6,**
**no. 50, 12 July 2017 (2017-07-12), XP055667546,**
**Retrieved from the Internet <URL:https://www.**
**mdpi.com/2304-8158/6/7/50> [retrieved on**
**20200123]**
• **VINCENT, D. ET AL.: "Quantitation and**
**identification of intact major milk proteins for**
**high-throughput LC-ESI-Q-TOF MS analyses",**
**PLOS ONE, vol. 11, no. 10, 2016, pages e0163471,**
**XP055596070, DOI: 10.1371/**
**journal.pone.0163471**
• **LUTTER, P. ET AL.: "Development and validation**
**of a method for the quantification of milk**
**proteins in food products based on liquid**
**chromatography with mass spectrometric**
**detection", JOURNAL OF AOAC**
**INTERNATIONAL, vol. 94, no. 4, 2011, pages 1043**
**- 59, XP055704579**

**(Cont. next page)**

- GIVENS, I. ET AL.: "Proportions of Al, A2, B and C beta-casein protein variants in retail milk in the UK", FOOD CHEMISTRY, vol. 139, no. 1-4, 2013, pages 549 - 52, XP055627672, DOI: 10.1016/j.foodchem.2013.01.115
- GIANSANTI, P. ET AL.: "Six alternative proteases for mass spectrometry-based proteomics beyond trypsin", NATURE PROTOCOLS, vol. 11, no. 5, 2016, pages 993 - 1006, XP055413585, DOI: 10.1038/nprot.2016.057
- GASTEIGER E ET AL.: "Protein Identification and Analysis Tools on the ExPASy Server", T HE PROTEOMICS PROTOCOLS HANDBOOK, 2005, Totowa NJ, pages 571 - 607, XP009163232, Retrieved from the Internet <URL:https://web.expasy.org/peptide_cutter> [retrieved on 20200205], DOI: 10.1385/1-59259-890-0:571

**Description**

**TECHNICAL FIELD**

The invention relates to the analysis of beta-casein proteins present in animal milk and dairy products prepared from animal milk. In particular, the invention relates to testing for the presence and quantification of A1-type beta-caseins and A2-type beta-caseins and related beta-casein variants in milk and milk derived dairy products.

**BACKGROUND OF THE INVENTION**

Milk, mainly bovine milk, consumed in populations throughout the world, is a major source of protein in human diets. Bovine milk typically comprises around 30 to 40 grams per litre of protein. Caseins make up the largest component (80%) of that protein, and beta-caseins make up about 37% of the caseins. In the past two decades the body of evidence implicating casein proteins, especially beta-caseins, in a number of physiological or biological actions (with some associated with health disorders) has been growing.

The beta-caseins can be categorised as A1-type beta-caseins and A2-type beta-caseins. A1 beta-casein and A2 beta-casein are the predominant beta-caseins in the bovine milk consumed in most human populations. A number of beta-casein variants can be categorised as A1-type or A2-type based on the amino acid residue at position 67 of the 209 amino acid sequence of beta-casein. A1 beta-casein differs from A2 beta-casein by a single amino acid. A histidine amino acid is located at position 67, whereas a proline is located at the same position of A2 beta-casein. This single amino acid difference is, however, critically important to the enzymatic digestion of beta-caseins in the gut. The presence of histidine at position 67 allows a protein fragment comprising seven amino acids, known as beta-casomorphin-7 (BCM-7), to be produced on enzymatic digestion. Thus, BCM-7 is a digestion product of A1 beta-casein. In the case of A2 beta-casein, position 67 is occupied by a proline which hinders cleavage of the amino acid bond at that location. Thus, BCM-7 is not a digestion product of A2 beta-casein.

Other beta-casein variants, such as the B, C, F, G and H1 variants, also have histidine at position 67, whereas the A3, D, E, H2 and I variants have proline at position 67 like the A2 variant. But these variants are found only in very low levels, or not found at all, in milk from cows of European origin. Thus, in the context of this invention, the term A1 beta-casein may refer to any beta-casein having histidine at position 67, and the term A2 beta-casein may refer to any beta-casein having proline at position 67.

BCM-7 is an opioid peptide and can bind to and activate opioid receptors throughout the body, and has been demonstrated to limit glutathione/antioxidant production leading to a change in cell function and gene expression.[1] BCM-7 has the ability to cross the gastrointestinal wall and enter circulation enabling it to influence systemic and cellular activities via opioid receptors. The applicant and others have previously determined an adverse association between the consumption of A1 beta-casein found in milk or milk products and type I diabetes,[2] coronary heart disease, neurological disorders,[4] inflammation of the bowel,[5] the symptoms of lactose intolerance,[6] the regulation of blood glucose levels,[7] cognitive function,[8] and gut microbiota.[9]

These reported findings all relate to the influence of BCM-7, or the beta-casein variants that produce BCM-7 upon digestion, on behavioural characteristics of individuals suffering from clinically diagnosed diseases or conditions. The findings do not relate to healthy individuals. Further, it is well-known that the outcome of studies based on cell culture experiments cannot reliably be extrapolated to *in vivo* or clinical effects since there are many variables relating to BCM-7 production, transport and metabolism that affect tissue exposure and therefore biological response. For example, it is known that the concentrations of opioids and their affinity to opioid receptors are important for cell response.

The growing body of scientific evidence linking beta-casein variants to adverse effects of consuming milk and dairy products has led to the importance of being able to reliably and accurately analyse milk and dairy products for the presence of beta-casein variants, particularly A1 beta-casein and its related variants having histidine at position 67 of the amino acid sequence.

Determination of the total levels of beta-casein in bovine milk is relatively trivial. The most common approach involves separation of the intact proteins by liquid chromatography (LC), followed by detection using either an ultraviolet detector (UV) or by mass spectrometry (MS). This approach was recently applied by Vincent *et al.*[10] to investigate milk in Australia, and by Givens *et al.*[11] to investigate milk in the United Kingdom. In both cases, the major beta-casein variants could be separately detected, but because purified variants of beta-casein are not available commercially as analytical standards, they could not be individually quantified. Moreover, both methods required the use of a high-resolution mass spectrometer, and neither are sensitive enough to distinguish trace levels of an A1 beta-casein variant in the presence of an excess of an A2 beta-casein variant.

An alternative approach involves digesting the milk proteins and quantifying the resulting peptides. This approach was adopted by Lutter *et al.*[12] who used trypsin to digest a range of foods to determine the concentrations of the major milk proteins. Samples were analysed by LC-MS/MS using internal calibration. While this method is more sensitive and more specific than intact protein methods, it lacks the critical ability to distinguish beta-casein variants.

While the above published methods maybe suited to the analysis of fresh milk, there is no method which satisfies all the necessary analytical requirements for modern single-variant milk products. This is especially true for products such as UHT milk or infant formula, where significant modification of the proteins can occur during processing. During analysis the caseins in these matrices may not behave in the same manner as 'wild type' caseins from fresh milk, and so analytical procedures must be adapted according to the matrix. For example, analysis of intact beta caseins from raw milk samples using LC-UV produces individual peaks for each beta casein variant which are suitable for both qualitative and quantitative analysis, as they can be easily identified and integrated. Conversely, the same procedure applied to infant formula yields broad peaks which are not resolved one another and are not appropriate for qualitative or quantitative analysis.

Analysis methods using digestion with proteolytic enzymes may also be subject to issues which prevent their application in analysis of beta casein. The most widely applied protease is trypsin, which when contacted with beta casein, produces a long peptide containing 48 residues. The substantial length of this peptide means that it is prone to multiple modifications, which results in a change of mass, which in turn substantially complicates detection using mass spectrometry. Additionally, the length makes this peptide difficult to synthesise for use as an analytical standard, and consequently, means that trypsin-based digestion methods are not appropriate for the quantitative analysis of beta casein in certain milk products. The use of alternative enzymes is thus necessary for beta casein analysis.

For example, CN 108519485 A refers to a mass spectrometric detection method of A1/A2 beta-casein using trypsin. The multi-step method comprises a) obtaining the amino acid sequence of A2 beta-casein; b) modifying the 67-site amino acid proline of A2 beta-casein to histidine, and obtaining the amino acid sequence of A1 beta-casein; and c) cutting a to-be-tested protein to a polypeptide segmented mixture with lower molecular mass using trypsin for enzymatic digestion, followed by several additional steps.

The applicant has now developed a method for the absolute determination of A1 beta-casein and A2 beta-casein variants in milk and milk products at commercially relevant levels.

It is therefore an object of the invention to provide a method for analysing beta-casein variants in animal milk and products derived from animal milk, or to at least provide a useful alternative to existing methods.

## SUMMARY OF THE INVENTION

In a first aspect of the invention there is provided a method for determining the amounts of A1-type beta-casein variants or A2-type beta-casein variants in a composition with greater than 80% accuracy, where the A1-type beta-casein variants are beta-casein variants having histidine at position 67 of the beta-casein amino acid sequence and the A2-type beta-casein variants are beta-casein variants having proline at position 67 of the beta-casein amino acid sequence, and where the composition is milk or a product prepared from milk, comprising the steps:

(i) contacting the composition with chymotrypsin under conditions enabling digestion of beta-caseins in the composition by the chymotrypsin;
(ii) obtaining an aqueous solution of beta-casein digestion peptides;
(iii) determining the concentrations of:

a) two or more beta-casein digestion peptides present in the solution, wherein each peptide comprises 8 to 20 amino acid residues and has histidine at a position corresponding to position 67 of the beta-casein amino acid sequence; and
b) two or more beta-casein digestion peptides present in the solution, wherein each peptide comprises 8 to 20 amino acid residues and has proline at a position corresponding to position 67 of the beta-casein amino acid sequence;

by analysing the mixture using liquid chromatography-mass spectrometry; and
(iv) calculating the concentration of A1-type beta-casein variants in the composition or the concentration of A2-type beta-casein variants in the composition using the concentrations of the beta-casein digestion peptides determined in step (iii).

In certain embodiments of the invention the A1-type beta-casein variants comprise any one or more of A1 beta-casein, B beta-casein, C beta-casein, F beta-casein, G beta casein and H1 beta-casein. For example, the A1-type beta-casein variants may comprise solely A1 beta-casein.

In certain embodiments of the invention the A2-type beta-casein variants comprise any one or more of A2 beta-casein, A3 beta-casein, D beta-casein, E beta-casein, H2 beta-casein and I beta-casein. For example, the A2-type beta-casein variants may comprise solely A2 beta-casein.

In some embodiments the A1-type beta-casein variants comprise solely A1 beta-casein and the A2-type beta-casein variants comprise solely A2 beta-casein.

In some embodiments of the invention the concentrations of three or four beta-casein digestion peptides having histidine at a position corresponding to position 67 of the beta-casein amino acid sequence are determined. In some embodiments, where the milk is bovine milk, the beta-casein digestion peptides are selected from the group comprising VYPFPGPIHN, SLVYPFPGPIHN, VYPFPGPIHNSLPQ, and SLVYPFPGPIHNSLPQ.

In some embodiments of the invention the concentrations of three or four beta-casein digestion peptides having proline at a position corresponding to position 67 of the beta-casein amino acid sequence are determined. In some embodiments, where the milk is bovine milk, the beta-casein digestion peptides are selected from the group comprising VYPFPGPIPN, SLVYPFPGPIPN, VYPFPGPIPNSLPQ, and SLVYPFPGPIPNSLPQ.

In certain embodiments of the invention the mixture analysed using liquid chromatography-mass spectrometry contains isotopically labelled peptides corresponding to each of the peptides from a) and b). The isotopically labelled peptides may be added to the composition before treatment with chymotrypsin in step (i) or after treatment with chymotrypsin in step (i).

In some embodiments of the invention where the milk is bovine milk the isotopically labelled peptides may correspond to the digestion peptides as follows:

| Digestion Peptide | Isotopically Labelled Peptide |
|---|---|
| VYPFPGPIHN | (*V)YPFPGPIHN |
| SLVYPFPGPIHN | SL(*V)YPFPGPIHN |
| VYPFPGPIHNSLPQ | VYPFPGPIHNS(*L)PQ |
| SLVYPFPGPIHNSLPQ | SL(*V)YPFPGPIHNSLPQ |

In some embodiments of the invention where the milk is bovine milk the isotopically labelled peptides may correspond to the digestion peptides as follows:

| Digestion Peptide | Isotopically Labelled Peptide |
|---|---|
| VYPFPGPIPN | (*V)YPFPGPIPN |
| SLVYPFPGPIPN | SL(*V)YPFPGPIPN |
| VYPFPGPIPNSLPQ | VYPFPGPIPNS(*L)PQ |
| SLVYPFPGPIPNSLPQ | SL(*V)YPFPGPIPNSLPQ |

In some embodiments of the invention where the milk is bovine milk the concentrations of the following peptides are determined in step (iii): VYPFPGPIHN, SLVYPFPGPIHN, VYPFPGPIHNSLPQ, SLVYPFPGPIHNSLPQ, VYPFPGPIPN, SLVYPFPGPIPN, VYPFPGPIPNSLPQ, and SLVYPFPGPIPNSLPQ.

In some embodiments of the invention the amounts of A1-type beta-casein variants and A2-type beta-casein variants in the composition are determined with greater than 90% accuracy, greater than 95% accuracy, or greater than 99% accuracy.

In some embodiments of the invention the composition is bovine milk or a milk product prepared from bovine milk.

In some embodiments of the invention the milk is fresh milk, raw milk, milk powder, liquid milk reconstituted from powder, skim milk, homogenised milk, condensed milk, evaporated milk, flavoured milk, UHT milk, pasteurised milk, or non-pasteurised milk. In some embodiments of the invention the milk product is infant formula, cream, yoghurt, quark, cheese, butter, or ice cream.

## BRIEF DESCRIPTION OF THE FIGURES

Figure 1 shows the proportion of A1-type beta-caseins in a series of relative milk standards analysed using the method of the invention, as well as intact HPLC analysis.

Figure 2 shows the amount of beta-casein in a series of milk samples and milk products as measured by the method of the invention, compared to the theoretical beta-casein level determined from the protein content of the samples.

## DETAILED DESCRIPTION

### *Definitions*

The term "beta-casomorphin-7" or "BCM-7" refers to the protein fragment Tyr-Pro-Phe-Pro-Gly-Pro-Ile, a heptapeptide produced on enzymatic digestion of bovine beta-casein variants that have a histidine, rather than a proline, at position 67 of the amino acid sequence.

The term "A1-type beta-casein variant" means any beta-casein variant having a histidine residue at position 67 of its amino acid sequence, and includes any one or more of A1 beta-casein, B beta-casein, C beta-casein, F beta-casein, and G beta casein.

The term "A2-type beta-casein variant" means any beta-casein variant having a proline residue at position 67 of its amino acid sequence, and includes A2 beta-casein, A3 beta-casein, D beta-casein, E beta-casein, H1 beta-casein, H2 beta-casein and I beta-casein.

The term "position 67" of the beta-casein amino acid sequence means the position numbered 67 counting amino acid residues of the sequence from the N terminus of the beta-casein (after removal of the signal peptide).

The term "AQ", sometimes referred to as "AQUA", means "absolute quantitation" and indicates that the method of the invention can be used to measure the absolute concentration of beta-caseins as a fraction of a sample (e.g. mass fraction), in contrast to other methods which do not measure absolute concentrations, but measure the proportion of beta-caseins relative to another component in a sample, or the proportion of one variant of beta-casein relative to another.

Throughout this specification, unless specifically stated otherwise or the context requires otherwise, reference to a single step, composition of matter, group of steps or group of compositions of matter shall be taken to encompass one and a plurality (i.e. one or more) of those steps, compositions of matter, groups of steps or group of compositions of matter.

It is intended that any reference to a range of numbers described in this specification (e.g., 1 to 10) also incorporates reference to all related numbers within that range (e.g., 1, 1.1, 2, 3, 3.9, 4, 5, 6, 6.5, 7, 8, 9 and 10) and also any range of rational numbers within that range (e.g., 2 to 8, 1.5 to 5.5 and 3.1 to 4.7) and therefore all sub-ranges of all ranges described in this specification are expressly disclosed. These are only examples of what is specifically intended and all possible combinations of numerical values between the lowest value and the highest value enumerated are to be considered to be expressly stated in this specification in a similar manner.

Throughout this specification the word "comprise", or variations such as "comprises" or "comprising", will be understood to imply the inclusion of a stated element, integer or step, or group of elements, integers or steps, but not the exclusion of any other element, integer or step, or group of elements, integers or steps.

Those skilled in the art will appreciate that the invention described in this specification is susceptible to variations and modifications other than those specifically described. The invention also includes all of the steps or features referred to or indicated in this specification, individually or collectively, and any and all combinations or any two or more of said steps or features.

The invention is not to be limited in scope by the specific examples described in this specification, which are intended for the purpose of exemplification only.

Any reference to prior art documents in this specification is not to be considered an admission that such prior art is widely known or forms part of the common general knowledge in the field.

### *Testing methodology*

The invention provides a method for determining the amounts of A1-type beta-casein variants and A2-type beta-casein variants in a composition. Importantly, the method enables the amounts of the two types of beta-casein variants to be determined with greater than 80% accuracy, and even up to 100% accuracy. The degree of accuracy has become critical as the demand for milk and milk products free of A1 beta-casein (and related variants) has increased dramatically in the past decade. Consumers as well as manufacturers and suppliers need to know whether or not a product is substantially free of A1-type beta-casein variants.

The method involves the digestion of beta-casein proteins in a composition with the serine protease enzyme chymotrypsin. The applicant has determined that the digestion process produces specific identifiable peptides. The presence and amounts of each peptide producing from the composition can be determined and the amounts then used to calculate the percentage amounts of A1-type beta-casein variants and A2-type beta-casein variants in the composition. The number of peptides (e.g., 2, 3, 4 or more) analysed determines the degree of accuracy. For example, analysis of only two peptides may produce a final determination of percentage amounts of the two types of beta-casein variants in the composition to an accuracy level of only 80%. Analysis of three peptides can produce an accuracy level of about 90%, and analysing for four peptides can produce an accuracy level of about 95%.

The composition to be analysed may be milk from any animal or a product prepared from animal milk. The composition may also be a non-milk product containing beta-casein that has been obtained from milk.

The milk may be in the form of fresh milk, raw milk, milk powder, liquid milk reconstituted from powder, skim milk, homogenised milk, condensed milk, evaporated milk, flavoured milk, UHT milk, pasteurised milk, or non-pasteurised milk, or any other form of milk.

The method of the invention is best suited for analysing bovine milk and bovine milk products, but can readily be adapted for milk and milk products from a wide variety of animals including humans, sheep, goats, horses, pigs, buffalo, deer, and any other mammals that produces caseins in their milk.

The composition may also be any milk-derived product such as infant formula, cream, yoghurt, quark, cheese, butter, or ice cream.

Where the composition to be analysed is milk, minimal sample preparation is needed. It is preferable to denature proteins contained in the milk sample, for example by treatment with a denaturing agent such as guanidine hydrochloride or urea.

In the case of milk products that are solid or semi-solid, sample preparation typically involves weighing an homogenised amount of the product into a separate vessel and extracting the casein proteins into a known volume of solution.

Digestion of the beta-caseins in the sample using chymotrypsin may be carried out under conditions suitable for enabling effective digestion. The sample is typically incubated at around 37 °C for several hours before quenching with a reagent, such as formic acid, to deactivate the chymotrypsin.

Once the sample has undergone chymotrypsin digestion, and any post-digestion sample preparation, certain isotopically labelled peptides are added to the sample prior to LC-MS (liquid chromatography - mass spectrum) analysis. The isotopically labelled peptides are selected based on the applicant's prior determination that chymotrypsin breaks down A1-type beta-casein variants to produce the digestion peptides:
VYPFPGPIHN, SLVYPFPGPIHN, VYPFPGPIHNSLPQ, and SLVYPFPGPIHNSLPQ and breaks down A2-type beta-casein variants to produce the digestion peptides:
VYPFPGPIPN, SLVYPFPGPIPN, VYPFPGPIPNSLPQ, and SLVYPFPGPIPNSLPQ

The isotopically labelled peptides may be from the group:
(*V)YPFPGPIHN, SL(*V)YPFPGPIHN, VYPFPGPIHNS(*L)PQ, SL(*V)YPFPGPIHNSLPQ, (*V)YPFPGPIPN, SL(*V)YPFPGPIPN, VYPFPGPIPNS(*L)PQ, and SL(*V)YPFPGPIPNSLPQ, where *V represents an isotopically labelled valine residue and *L represents an isotopically labelled leucine residue. It should be appreciated that any, or multiple, amino acid residues of a peptide may be isotopically labelled. However, isotopically labelled valine or leucine are generally preferred.

In some embodiments of the invention, the isotopically labelled peptides used correspond to the digestion peptides according to Table 1.

**Table 1: Digestion peptides and their isotopically labelled equivalents**

| Digestion Peptide | Isotopically Labelled Peptide |
|---|---|
| VYPFPGPIHN | (*V)YPFPGPIHN |
| SLVYPFPGPIHN | SL(*V)YPFPGPIHN |
| VYPFPGPIHNSLPQ | VYPFPGPIHNS(*L)PQ |
| SLVYPFPGPIHNSLPQ | SL(*V)YPFPGPIHNSLPQ |
| VYPFPGPIPN | (*V)YPFPGPIPN |
| SLVYPFPGPIPN | SL(*V)YPFPGPIPN |
| VYPFPGPIPNSLPQ | VYPFPGPIPNS(*L)PQ |
| SLVYPFPGPIPNSLPQ | SL(*V)YPFPGPIPNSLPQ |

The method of the invention requires adding at least two of the above isotopically labelled peptides to the sample. However, for greater accuracy, three, four, or possibly more, isotopically labelled peptides are added to the sample.

LC-MS/MS enables ready identification of the presence of any of the digestion peptides based on the retention time of the peptides and the ion ratios of the fragments produced when analysed in MS/MS mode. This is alternately referred to as multiple reaction monitoring (MRM) mode, or selected reaction monitoring (SRM) mode. Comparison of the responses of each peptide to an isotopically labelled internal standard peptide present in the mixture and to a concurrently run standard curve allows the concentration of each peptide to be determined, from which the concentrations of the A1-type beta-casein variants in the composition and the A2-type beta casein variants in the composition can be calculated.

*Discussion of the Examples*

Examples of the experimental steps are provided below. Example 1 shows how samples may be prepared for analysis. This generally includes the extraction of beta-caseins into an aqueous state, wherein they can be unfolded by the action of a chaotropic denaturing agent such urea, so that the later action of chymotrypsin on the sample is more efficient. In the case of liquid samples such as whole milk or UHT milk, this simply involves the transfer of an aliquot of the sample to a vessel and the addition of the denaturing agent. For solid or semi-solid samples, this involves the preparation of an accurately defined solution of the solid dissolved in an extraction buffer, typically an aqueous buffer. Subsequently, the samples are treated in the same manner as for liquid samples.

Example 2 describes the chymotrypsin digestion step. The denatured samples are exposed to a solution of chymotrypsin under carefully controlled conditions. These conditions typically include a buffer to control the pH of the solution, as proteolytic enzymes are only active within a narrow pH range. Conditions typically also include mechanical agitation of the digest to reduce or eliminate mass transfer limitations, and heat to accelerate the reaction. This results in a controlled cleavage of the beta-caseins, and other proteins within the matrix, to yield a set of peptides which can be uniquely identified as originating from a specific protein. After a pre-determined period of time the reaction is halted ('quenched') by the addition of a concentrated acid solution in organic solvent, which immediately stops the digestion. The samples are then fortified by the addition of isotopically-labelled internal standard peptides which are subsequently used for calculating the concentrations of the beta-caseins.

Example 3 describes the analysis of samples using liquid chromatography with triple quadrupole mass spectrometric detection. This results in spatial and temporal separation of the peptides from one another, which can be individually quantified by passage of the eluent to a mass spetrometer operated in multiple reaction monitoring mode, or an equivalently specific detection procedure such as high resolution selected ion monitoring. The peak areas of target peptides are determined by integration, as well as the isotopically-labelled internal standards. The peak area ratios are then used to determine the concentration of the target peptides by comparision against a standard curve. To ensure the peptides are correctly identified, quality control criteria such retention time matching and ion ratio matching are employed in the procedure.

In Example 4, the concentrations of the peptides are used to calculate the concentrations of the parent beta-caseins. In general, conversion factors are derived based on the ratio of the mass of the peptide to the mass of the parent protein, and are used to convert from concentration of peptide to concentration of protein. As the target peptides have overlapping sequences, the concentrations can then be summed to find the total A1-type beta-casein content, and the total A2-type beta-casein content, as well as figures derived from these values, such as the proportion of A1-type beta-casein in the sample relative to the total beta-casein.

Example 5 shows application of the method to the analysis of a range of milk products. The results are compared and contrasted with results from existing methods for determining the percentage of A1-type beta-caseins in a sample of milk or a milk product. For a range of matrix types, it is shown that the concentration of beta-caseins in the sample is consistent with the expected values based on the labelled protein content, and thus the proportion of A1-type beta-caseins in the samples is correct. Morover, the proportion of A1-type beta-caseins is consistent with previous methods for simple matrices like milk or milk powder, but provides a more accurate estimate for heavily modified products such as infant formula. The results are shown in Table 6.

Column 1 of Table 6 indicates the identity of the products analysed. Column 2 shows the total protein content of each sample as determined by Kjeldahl nitrogen analysis. This may be compared to column 3, which shows the protein content as specified on the label of the product. In general there is good agreement between these two measures, indicating that the labelled protein content is accurate. Column 4 shows the theoretical beta-casein content of each sample, determined by multiplication of the labelled protein value by 0.32, a commonly accepted conversion factor representing the mass fraction of beta-casein in milk products relative to the total protein content.

Column 9 shows the total beta-casein content, expressed in g/100g, as the sum of A1-type beta-caseins and A2-type beta-caseins, as determined by the method of the invention. These show generally good agreement with the values in column 4, indicating that the method is accurate and fit for purpose.

Column 10 shows the percentage of A1-type beta-caseins of the total beta-casein content, calculated as the sum of A1-type beta-caseins and A2-type beta-caseins. These results may be compared to the results in column 5, which presents the %A1 as determined by intact protein analysis using high performance liquid chromatography with ultraviolet detection. For milk and milk products which are not significantly modified, there is good agreement between these two figures. However, there are significant discrepancies in the case of infant formula samples, which by nature of their manufacture, are unsuitable for intact analysis and produce erroneous results. Similar trends are seen when Column 10 is compared to column 6, which shows the percentage of A1-type beta-caseins determined using a primitive enzymatic method utilising a tryptic digestion of samples with subsequent calibration against volumetrically-diluted relative milk standards.

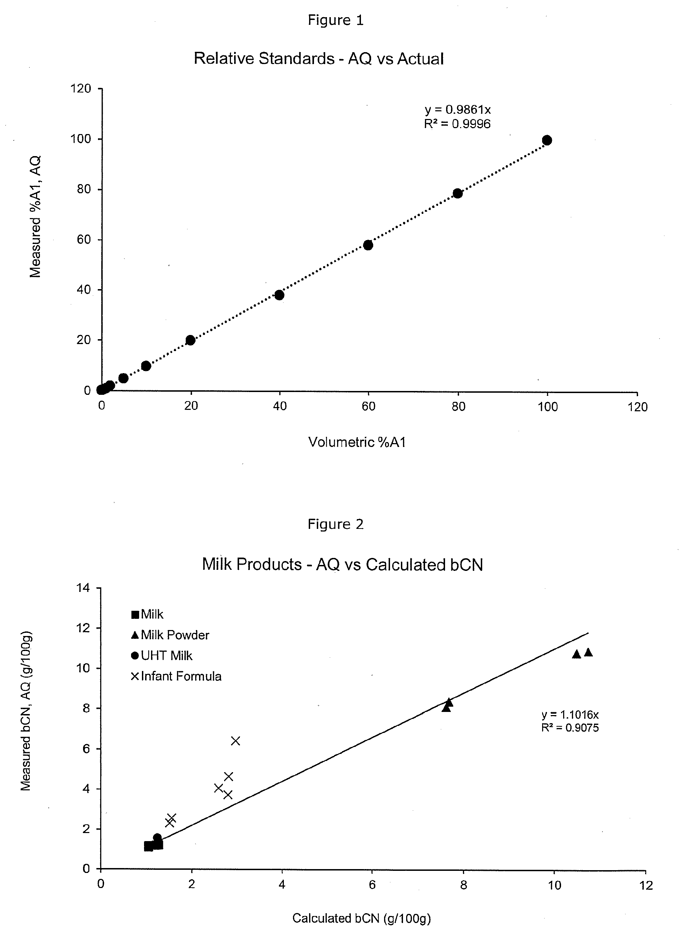

Figure 1 shows the proportion of A1-type beta-caseins in a series of relative milk standards analysed using the method of the invention, as well as intact HPLC analysis. The samples were created by volumetric dilution of milk from two individual animals, one verified to be of the A1/A1 genotype and thus producing milk containing pure A1-type beta-casein, and the other of the A2/A2 genotype thus producing milk containing pure A2-type beta-casein. Tight clustering of data points around the best fit line indicates the current method is precise, as indicated by the correlation coefficient ($R^2$ =

0.9996). The slope of the line (0.9861) is interpreted to indicate the current method is also accurate.

Figure 2 shows the amount of beta-casein in a series of milk samples and milk products as measured by the method of the invention, compared to the theoretical beta-casein level determined from the protein content of the samples. The good clustering of data points about the best fit line indicates the method is precise, also indicated by the correlation coefficient ($R^2$ = 0.9075). The slope of the line (0.9075) also indicates the method is accurate.

The invention is further described with reference to the following examples. It will be appreciated that the invention as claimed is not intended to be limited in any way by the examples.

## EXAMPLES

### Example 1: Sample preparation

*Liquid samples* - For non-viscous liquid samples, e.g. milk, the liquid (40 μL) was transferred to a 2 mL centrifuge tube. Urea solution (120 μL, 500 g/L) was added to denature proteins, and the mixture then mixed at 2000 RPM for 5 min.

*Solid samples* - For solid samples or viscous liquid samples, the sample (5.00 ±0.01 g) was weighed into a falcon tube (50 ml). Ammonium bicarbonate solution (46.6 ml, 4 g/L) was added and the mixture shaken to wet all the powder. After incubation at 50 °C for 15 min, the mixture was shaken until homogeneous. In the case of infant formula, a sample (100 μL) was transferred to a 2 mL centrifuge tube, casein precipitant (150 g/L ammonium acetate pH 4.2, 1000 μL) was added and the mixture mixed at 2000 RPM for 5 min and then centrifuged at 5000 RCF for 5 min. After decanting the supernatant, casein precipitant (1000 μL) was added to the solid pellet and the mixture mixed at 2000 RPM for 5 min and then centrifuged at 5000 RCF for 5 min before decanting the supernatant. Water (1000 μL) was added to the solid pellet, the mixing and centrifuging steps repeated and the supernatant decanted. Urea solution (120 μL, 500 g/L) was added to denature proteins, and the mixture then mixed at 2000 for 5 min.

### Example 2: Chymotrypsin digestion

Chymotrypsin solution (1000 μL) was added to a sample prepared in accordance with Example 1. The mixture was incubated at 37 °C for 8 hours while shaking at 200 RPM. Quench solution (120 μL, 500 g/L formic acid solution) was added and the mixture mixed at 2000 RPM for 5 min and centrifuged at 10,000 RCF for 10 min. Sample (980 μL) was transferred to an LC vial, a solution of isotopically labelled peptide standards (20 μL) was added, and the mixture vortexed for 10 sec. The solution of labelled peptide standards (Biomatik, custom order, ≥95.0%) was prepared by weighing each of the peptides (2.00 ±0.10 mg) in Table 1 into a 2 mL polyethylene centrifuge tube. The peptides were dissolved by adding of 20% acetonitrile containing 0.1% formic acid. The volume was adjusted to obtain a concentration of 1,000.0 mg/L. For example, if only 1.90 mg was weighed, then 1.90 mL of solution was added. The labelled peptide standards of Table 2 correspond with the target peptides of Table 3.

*Table 2: Isotopically labelled peptide standards*

| Peptide | Sequence | Formula | [M+H]⁺ Ion (m/z) |
|---|---|---|---|
| A1C-VN(10)-H | (*V)YPFPGPIHN | $[^{13}C_5,^{15}N_1]$-$C_{51}H_{77}N_{11}O_{13}$ | 1146.32 |
| A1C-SN(12)-H | SL(*V)YPFPGPIHN | $[^{13}C_5,^{15}N_1]$-$C_{60}H_{93}N_{14}O_{16}$ | 1346.56 |
| A1C-VQ(14)-H | VYPFPGPIHNS(*L)PQ | $[^{13}C_6,^{15}N_1]$-$Ce_9H_{10}sN_{17}O_{19}$ | 1572.81 |
| A1C-SQ(16)-H | SL(*V)YPFPGPIHNSLPQ | $[^{13}C_5,5N_1]$-$C_{79}H_{124}N_{19}O_{22}$ | 1772.16 |
| A2C-VN(10)-H | (*V)YPFPGPIPN | $[^{13}C_5,^{15}N,]$-$C_{50}H_{77}N_{10}O_{13}$ | 1106.29 |
| A2C-SN(12)-H | SL(*V)YPFPGPIPN | $[^{13}C_5,^{15}N,]$-$C_{59}H_{93}N_{12}O_{16}$ | 1306.53 |
| A2C-VQ(14)-H | VYPFPGPIPNS(*L)PQ | $[^{13}C_6,^{15}N_1]$-$C_{68}H_{108}N_{15}O_{19}$ | 1532.78 |
| A2C-SQ(16)-H | SL(*V)YPFPGPIPNSLPQ | $[^{13}C_5,^{15}N_1]$-$C_{78}H_{124}N_{17}O_{22}$ | 1732.02 |

*Table 3: Target peptides*

| Peptide | Sequence | Formula | [M+H]⁺ Ion (m/z) |
|---|---|---|---|
| A1C-VN(10) | VYPFPGPIHN | $C_{56}H_{77}N_{13}O_{13}$ | 1140.28 |
| A1C-SN(12) | SLVYPFPGPIHN | $C_{65}H_{93}N_{15}O_{16}$ | 1340.65 |
| A1C-VQ(14) | VYPFPGPIHNSLPQ | $C_{75}H_{108}N_{18}O_{19}$ | 1565.81 |
| A1C-SQ(16) | SLVYPFPGPIHNSLPQ | $C_{84}H_{124}N_{20}O_{22}$ | 1766.16 |
| A2C-VN(10) | VYPFPGPIPN | $C_{55}H_{77}N_{11}O_{13}$ | 1100.39 |

(continued)

| Peptide | Sequence | Formula | [M+H]+ Ion (m/z) |
|---|---|---|---|
| A2C-SN(12) | SLVYPFPGPIPN | $C_{64}H_{93}N_{13}O_{16}$ | 1300.62 |
| A2C-VQ(14) | VYPFPGPIPNSLPQ | $C_{74}H_{108}N_{16}O_{19}$ | 1525.78 |
| A2C-SQ(16) | SLVYPFPGPIPNSLPQ | $C_{83}H_{124}N_{18}O_{22}$ | 1726.13 |

### Example 3: LC-MS/MS analysis

Samples (1 μL) were injected onto a Waters Xselect HSS T3 C18 column (2.5 um x 2.1 mm x 50 mm) with attached Phenomenex KrudCatcher using a Thermo Vanquish LC system. Separation parameters are summarised in Table 4. Briefly, a gradient was run from 20% acetonitrile up to 40% acetonitrile to separate the target peptides, during which point the eluent was passed to a mass spectrometer for detection. The column was then washed at elevated flow prior to commencing the next injection. During this wash period the unwanted components of the sample were diverted to waste, and the acquisition turned off.

*Table 4: Gradient and selected acquisition parameters*

| Time | %A | %B | Flow | Divert | MS Acquisition |
|---|---|---|---|---|---|
| 0.0 | 80 | 20 | 0.5 | 1-6 | Off |
| 0.5 | 80 | 20 | 0.5 | 1-2 | On |
| 4.0 | 60 | 40 | 0.5 | 1-2 | Off |
| 4.5 | 0 | 100 | 0.8 | 1-6 | Off |
| 6.0 | 0 | 100 | 0.8 | 1-6 | Off |
| 6.5 | 80 | 20 | 0.8 | 1-6 | Off |
| 7.5 | 80 | 20 | 0.5 | 1-6 | Off |
| 8.0 | 80 | 20 | 0.5 | 1-6 | Off |

Mass spectral acquisition was performed using a Thermo TSQ Altis Mass Spectrometer, operating in position ion MRM mode. Each peptide has both quantifier and qualifier transitions, resulting in a total of 32 MRMs. These were all monitored continuously over the acquisition period, without the use of scheduling. The dwell time was set to 10 ms, the Q1 and Q3 resolutions to 2 Da, and the CID gas to 2 mTorr. A list of the transitions and associated parameters is shown in Table 5.

*Table 5: MRM data for the sixteen target peptides monitored method*

| Designation | Transition(s) | Usage | CE (V) | Rf (V) |
|---|---|---|---|---|
| A1C-VN(10) | 571.2 → 440.1 | Quantifier | 16 | 60 |
| | 571.2 → 634.3 | Qualifier | 22 | 60 |
| A1C-SN(12) | 671.2 → 878.6 | Quantifier | 21 | 60 |
| | 671.2 → 300.2 | Qualifier | 22 | 60 |
| A1C-VQ(14) | 783.9 → 244.4 | Quantifier | 27 | 70 |
| | 783.9 → 652.5 | Qualifier | 22 | 70 |
| A1C-SQ(16) | 883.5 → 244.4 | Quantifier | 28 | 70 |
| | 883.5 → 1523.1 | Qualifier | 25 | 70 |
| A1C-VN(10)-H | 574.2 → 440.1 | Quantifier | 16 | 60 |
| | 574.2 → 634.3 | Qualifier | 22 | 60 |
| A1C-SN(12)-H | 674.3 → 878.6 | Quantifier | 21 | 60 |
| | 674.3 → 306.2 | Qualifier | 22 | 60 |
| A1C-VQ(14)-H | 787.4 → 244.4 | Quantifier | 27 | 70 |
| | 787.4 → 656.0 | Qualifier | 22 | 70 |
| A1C-SQ(16)-H | 886.8 → 244.4 | Quantifier | 28 | 70 |
| | 886.8 → 1523.1 | Qualifier | 25 | 70 |
| A2C-VN(10) | 1100.6 → 594.3 | Quantifier | 39 | 60 |
| | 1100.6 → 838.4 | Qualifier | 37 | 60 |

(continued)

| Designation | Transition(s) | Usage | CE (V) | Rf (V) |
|---|---|---|---|---|
| A2C-SN(12) | 1300.7 → 594.4 | Quantifier | 42 | 60 |
| | 1300.7 → 838.4 | Qualifier | 42 | 60 |
| A2C-VQ(14) | 763.4 → 244.2 | Quantifier | 20 | 70 |
| | 763.4 → 655.4 | Qualifier | 19 | 70 |
| A2C-SQ(16) | 863.5 → 244.2 | Quantifier | 24 | 70 |
| | 863.5 → 655.3 | Qualifier | 20 | 70 |
| A2C-VN(10)-H | 1107.0 → 594.3 | Quantifier | 39 | 60 |
| | 1107.0 → 838.4 | Qualifier | 37 | 60 |
| A2C-SN(12)-H | 1307.1 → 594.4 | Quantifier | 42 | 60 |
| | 1307.1 → 838.4 | Qualifier | 42 | 60 |
| A2C-VQ(14)-H | 767.4 → 244.2 | Quantifier | 20 | 70 |
| | 767.4 → 662.4 | Qualifier | 19 | 70 |
| A2C-SQ(16)-H | 867.0 → 244.2 | Quantifier | 24 | 70 |
| | 867.0 → 655.3 | Qualifier | 20 | 70 |

### Example 4: Concentration determinations for A1-type beta-casein variants and A2-type beta-casein variants

Calculations were performed automatically using a pre-designed digital spreadsheet, though they can be done manually. The concentration of A1-beta-casein variants was calculated by summing the products of the concentration of each peptide by the quotient of the parent protein mass and the peptide mass, then multiplying by the preparation factor:

$$C_{A1-\beta CN} = \left( \left( C_{A1C-VN(10)} \times \frac{Mw_{A1-\beta CN}}{Mw_{A1C-VN(10)}} \right) + \left( C_{A1C-SN(12)} \times \frac{Mw_{A1-\beta CN}}{Mw_{A1C-SN(12)}} \right) \right.$$
$$\left. + \left( C_{A1C-VQ(14)} \times \frac{Mw_{A1-\beta CN}}{Mw_{A1C-VQ(14)}} \right) + \left( C_{A1C-SQ(16)} \times \frac{Mw_{A1-\beta CN}}{Mw_{A1C-SQ(16)}} \right) \right) \times P$$

The same calculation was used for calculating the concentration of A2-beta-casein variants, but with masses adjusted accordingly:

$$C_{A2-\beta CN} = \left( \left( C_{A2C-VN(10)} \times \frac{Mw_{A2-\beta CN}}{Mw_{A2C-VN(10)}} \right) + \left( C_{A2C-SN(12)} \times \frac{Mw_{A2-\beta CN}}{Mw_{A2C-SN(12)}} \right) \right.$$
$$\left. + \left( C_{A2C-VQ(14)} \times \frac{Mw_{A2-\beta CN}}{Mw_{A2C-VQ(14)}} \right) + \left( C_{A2C-SQ(16)} \times \frac{Mw_{A2-\beta CN}}{Mw_{A2C-SQ(16)}} \right) \right) \times P$$

The preparation factor, P, was calculated as follows:

$$P = \frac{v_1}{m_1} \times \frac{v_2}{v_3} \times \frac{v_4}{v_5}$$

where $v_1$ is the final volume of the product after extraction, $m_1$ is the mass of product weighed, $v_2$ is the volume in the digestion tube after quenching, $V_3$ is the volume of extract added to the digestion tube, $V_4$ is the final volume in the LC vial, and $V_5$ is the volume of digest transferred to the LC vial.

The total beta-casein concentration was calculated by summing the concentrations of the two polymorphs:

$$C_{Total \, \beta CN} = C_{A1 \beta CN} + C_{A2 \beta CN}$$

The percentage of A1-beta-casein variants in the product was calculated by dividing the A1-beta-casein variants concentration by the sum of the A1- and A2-beta-casein variants concentrations:

**11**

$$\%A1 = \frac{C_{A1\beta CN}}{C_{A1\beta CN} + C_{A2\beta CN}} \times 100\%$$

### Example 5: Milk and milk product analysis

Various fresh milk, milk powder, infant formula and UHT milk samples were tested according to the method outlined in Examples 1-4. A series of milk standards were also prepared and tested. The results are shown in Table 4, wherein results determined according to the method of the invention are compared to other methods for the determination of beta-casein content.

*Table 6: Concentration data for beta-casein variants in milk and milk products*

| Sample | Kjeldahl Protein (g/100g) | Labelled Protein (g/100g) | Calculated bCN (g/100g) | Intact UV % A1 | Tryptic %A1 | AQ A1 (g/100g) | AQ A2 (g/100g) | AQ Total bCN (g/100g) | AQ % A1 |
|---|---|---|---|---|---|---|---|---|---|
| Milk (non-homogenised, A1/A2) | 3.62 | 3.80 | 1.22 | 39.79 | 18.26 | 0.34 | 0.84 | 1.18 | 28.77 |
| Milk (full fat, A1/A2) | 3.17 | 3.30 | 1.06 | 40.93 | 18.10 | 0.03 | 0.07 | 1.09 | 28.59 |
| Milk (reduced fat, A1/A2) | 3.09 | 3.30 | 1.06 | 42.04 | 19.28 | 0.35 | 0.80 | 1.15 | 30.26 |
| Milk (fat-free, A1/A2) | 3.57 | 4.00 | 1.28 | 38.53 | 18.54 | 0.34 | 0.85 | 1.19 | 28.52 |
| Milk Powder (full fat, A2) | 21.3 | 23.80 | 7.62 | 0.00 | 0.00 | 0.01 | 8.11 | 8.12 | 0.13 |
| Milk Powder (reduced fat, A2) | 29.9 | 33.60 | 10.75 | 0.00 | 0.00 | 0.02 | 10.89 | 10.90 | 0.15 |
| Milk Powder (full fat, A1/A2) | 21.8 | 24.00 | 7.68 | 35.83 | 16.03 | 2.29 | 6.09 | 8.39 | 27.35 |
| Milk Powder (reduced fat, A1/A2) | 29.3 | 32.80 | 10.50 | 34.77 | 16.78 | 3.10 | 7.70 | 10.81 | 28.71 |
| Infant Formula (Stage 1, A2) | 9.41 | 10.15 | 1.51 | 28.24 | 0.00 | 0.18 | 2.15 | 2.33 | 7.64 |
| Infant Formula (Stage 2, A2) | 13.3 | 15.25 | 2.82 | 32.44 | 0.00 | 0.14 | 4.52 | 4.66 | 2.94 |
| Infant Formula (Stage 3, A2) | 12.8 | 13.91 | 2.60 | 38.32 | 0.67 | 0.18 | 3.89 | 4.07 | 4.52 |
| Infant Formula (Stage 1, A1/A2) | 9.6 | 10.55 | 1.56 | 39.56 | 14.02 | 0.72 | 1.85 | 2.57 | 28.19 |
| Infant Formula (Stage 2, A1/A2) | 13.3 | 15.03 | 2.80 | 32.79 | 14.67 | 1.08 | 2.67 | 3.74 | 28.71 |
| Infant Formula (Stage 3, A1/A2) | 15.7 | 16.10 | 2.97 | 43.00 | 23.37 | 2.19 | 4.25 | 6.44 | 34.02 |
| UHT Milk (reduced fat, A1/A2) | 4.82 | 3.90 | 1.25 | 42.96 | 18.62 | 0.44 | 1.12 | 1.57 | 28.38 |
| UHT Milk (full fat, A1/A2) | 3.73 | 4.00 | 1.28 | 39.20 | 18.14 | 0.39 | 0.91 | 1.30 | 30.12 |

(continued)

| Sample | Kjeldahl Protein (g/100g) | Labelled Protein (g/100g) | Calculated bCN (g/100g) | Intact UV % A1 | Tryptic %A1 | AQ A1 (g/100g) | AQ A2 (g/100g) | AQ Total bCN (g/100g) | AQ % A1 |
|---|---|---|---|---|---|---|---|---|---|
| Relative milk standard (0.0% A1) | - | - | - | 0.14 | 0.00 | 0.00 | 1.51 | 1.52 | 0.16 |
| Relative milk standard (0.5% A1) | - | - | - | 0.44 | 0.43 | 0.01 | 1.54 | 1.55 | 0.48 |
| Relative milk standard (1.0% A1) | - | - | - | 0.96 | - | 0.01 | 1.45 | 1.47 | 0.92 |
| Relative milk standard (2.0% A1) | - | - | - | 1.73 | 2.55 | 0.03 | 1.45 | 1.48 | 1.91 |
| Relative milk standard (5.0% A1) | - | - | - | 4.60 | 4.41 | 0.07 | 1.45 | 1.52 | 4.87 |
| Relative milk standard (10.0% A1) | - | - | - | 9.68 | 9.38 | 0.15 | 1.41 | 1.56 | 9.80 |
| Relative milk standard (20.0% A1) | - | - | - | 20.93 | 20.93 | 0.30 | 1.22 | 1.52 | 20.00 |
| Relative milk standard (40.0% A1) | - | - | - | 40.01 | 37.86 | 0.57 | 0.94 | 1.51 | 37.99 |
| Relative milk standard (60.0% A1) | - | - | - | 59.88 | 64.97 | 0.81 | 0.59 | 1.39 | 58.02 |
| Relative milk standard (80.0% A1) | - | - | - | - | - | 1.08 | 0.29 | 1.37 | 78.76 |
| Relative milk standard (100% A1) | - | - | - | 98.58 | - | 1.41 | 0.00 | 1.41 | 99.93 |

**REFERENCES**

1. Trivedi, M.S., Shah, J.S., Al-Mughairy, S., Hodgson, N.W., Simms, B., Trooskens, G., Van Criekinge, W., and Deth, R.C., J. Nutr. Biochem., 2014, 25, 1011-1018.
2. WO 1996/014577
3. WO 1996/036239
4. WO 2002/019832
5. WO 2014/193248
6. WO 2015/005804
7. WO 2015/026245
8. WO 2017/171563
9. WO 2018/063008

10. Vincent, D., Elkins, A., Condina, M.R., Ezernieks, V., and Rochfort, S., 2016, PLOS ONE 11, e0163471.

11. Givens, I., Aikman, P., Gibson, T., and Brown, R., Food Chemistry, 2013, 139, 549-552.

12. Lutter, P., Parisod, V., and Weymuth, H., J. AOAC Int., 2011, 94, 1043-1059.

**Claims**

1. A method for determining the amounts of A1-type beta-casein variants or A2-type beta-casein variants in a composition with greater than 80% accuracy, where the A1-type beta-casein variants are beta-casein variants having histidine at position 67 of the beta-casein amino acid sequence and the A2-type beta-casein variants are beta-casein variants having proline at position 67 of the beta-casein amino acid sequence, and where the composition is milk or a product prepared from milk, comprising the steps:

   (i) contacting the composition with chymotrypsin under conditions enabling digestion of beta-caseins in the composition by the chymotrypsin;
   (ii) obtaining an aqueous solution of beta-casein digestion peptides;
   (iii) determining the concentrations of:

      a) two or more beta-casein digestion peptides present in the solution, wherein each peptide comprises 8 to 20 amino acid residues and has histidine at a position corresponding to position 67 of the beta-casein amino acid sequence; and
      b) two or more beta-casein digestion peptides present in the solution, wherein each peptide comprises 8 to 20 amino acid residues and has proline at a position corresponding to position 67 of the beta-casein amino acid sequence;

   by analysing the mixture using liquid chromatography-mass spectrometry; and
   (iv) calculating the concentration of A1-type beta-casein variants in the composition or the concentration of A2-type beta-casein variants in the composition using the concentrations of the beta-casein digestion peptides determined in step (iii).

2. A method as claimed in claim 1, wherein the A1-type beta-casein variants comprise solely A1 beta-casein.

3. A method as claimed in claim 1, wherein the A2-type beta-casein variants comprise solely A2 beta-casein.

4. A method as claimed in any one of claims 1 to 3, wherein the concentrations of three or four beta-casein digestion peptides having histidine at a position corresponding to position 67 of the beta-casein amino acid sequence are determined.

5. A method as claimed in claim 4, wherein the milk is bovine milk and the beta-casein digestion peptides are selected from the group comprising:

   (i) VYPFPGPIHN;
   (ii) SLVYPFPGPIHN;
   (iii) VYPFPGPIHNSLPQ; and
   (iv) SLVYPFPGPIHNSLPQ.

6. A method as claimed in any one of claims 1 to 3, wherein the concentrations of three or four beta-casein digestion peptides having proline at a position corresponding to position 67 of the beta-casein amino acid sequence are determined.

7. A method as claimed in claim 6, wherein the milk is bovine milk and the beta-casein digestion peptides are selected from the group comprising:

   (i) VYPFPGPIPN;
   (ii) SLVYPFPGPIPN;
   (iii) VYPFPGPIPNSLPQ; and
   (iv) SLVYPFPGPIPNSLPQ.

8. A method as claimed in any one of claims 1 to 7, wherein the mixture analysed using liquid chromatography-mass spectrometry contains isotopically labelled peptides corresponding to each of the peptides from a) and b) of step (iii).

9. A method as claimed in claim 8, wherein the isotopically labelled peptides correspond to the digestion peptides as follows:

| Digestion Peptide | Isotopically Labelled Peptide |
|---|---|
| VYPFPGPIHN | (*V)YPFPGPIHN |
| SLVYPFPGPIHN | SL(*V)YPFPGPIHN |
| VYPFPGPIHNSLPQ | VYPFPGPIHNS(*L)PQ |
| SLVYPFPGPIHNSLPQ | SL(*V)YPFPGPIHNSLPQ |

10. A method as claimed in claim 8, wherein the isotopically labelled peptides correspond to the digestion peptides as follows:

| Digestion Peptide | Isotopically Labelled Peptide |
|---|---|
| VYPFPGPIPN | (*V)YPFPGPIPN |
| SLVYPFPGPIPN | SL(*V)YPFPGPIPN |
| VYPFPGPIPNSLPQ | VYPFPGPIPNS(*L)PQ |
| SLVYPFPGPIPNSLPQ | SL(*V)YPFPGPIPNSLPQ |

11. A method as claimed in claim 1, wherein the milk is bovine milk and the concentrations of the following peptides are determined in step (iii):

(i) VYPFPGPIHN;
(ii) SLVYPFPGPIHN;
(iii) VYPFPGPIHNSLPQ;
(iv) SLVYPFPGPIHNSLPQ;
(v) VYPFPGPIPN;
(vi) SLVYPFPGPIPN;
(vii) VYPFPGPIPNSLPQ; and
(viii) SLVYPFPGPIPNSLPQ.

12. A method as claimed in any one of claims 1 to 11, wherein the amounts of A1-type beta-casein variants and A2-type beta-casein variants in the composition are determined with greater than 90% accuracy.

13. A method as claimed in any one of claims 1 to 12, wherein the amounts of A1-type beta-casein variants and A2-type beta-casein variants in the composition are determined with greater than 95% accuracy.

14. A method as claimed in any one of claims 1 to 13, wherein the amounts of A1-type beta-casein variants and A2-type beta-casein variants in the composition are determined with greater than 99% accuracy.

15. A method as claimed in any one of claims 1 to 14, wherein the composition is bovine milk or a milk product prepared from bovine milk.

**Patentansprüche**

1. Verfahren zum Bestimmen der Mengen von Beta-Casein-Varianten vom Typ A1 oder Beta-Casein-Varianten vom Typ A2 in einer Zusammensetzung mit einer Genauigkeit von über 80 %, wobei die Beta-Casein-Varianten vom Typ A1 Beta-Casein-Varianten sind, die Histidin an Position 67 der Beta-Casein-Aminosäuresequenz aufweisen, und die Beta-Casein-Varianten vom Typ A2 Beta-Casein-Varianten sind, die Prolin an Position 67 der Beta-Casein-Aminosäuresequenz aufweisen, und wobei die Zusammensetzung Milch oder ein aus Milch hergestelltes Produkt ist, umfassend die Schritte:

(i) In-Verbindung-Bringen der Zusammensetzung mit Chymotrypsin unter Bedingungen, die eine Verdauung von Beta-Caseinen in der Zusammensetzung durch das Chymotrypsin ermöglichen;
(ii) Erhalten einer wässrigen Lösung von Beta-Casein-Verdauungspeptiden;
(iii) Bestimmen der Konzentrationen von:

a) zwei oder mehr in der Lösung vorhandenen Beta-Casein-Verdauungspeptiden, wobei jedes Peptid 8 bis 20 Aminosäurereste umfasst und Histidin an einer Position aufweist, die Position 67 der Beta-Casein-Aminosäuresequenz entspricht; und
b) zwei oder mehr in der Lösung vorhandenen Beta-Casein-Verdauungspeptiden, wobei jedes Peptid 8 bis 20 Aminosäurereste umfasst und Prolin an einer Position aufweist, die Position 67 der Beta-Casein-Aminosäuresequenz entspricht;

durch Analysieren der Mischung unter Verwendung von Flüssigkeitschromatographie-Massenspektrometrie; und
(iv) Berechnen der Konzentration von Beta-Casein-Varianten vom Typ A1 in der Zusammensetzung oder der Konzentration von Beta-Casein-Varianten vom Typ A2 in der Zusammensetzung unter Verwendung der in Schritt (iii) bestimmten Konzentrationen der Beta-Casein-Verdauungspeptide.

2. Verfahren nach Anspruch 1, wobei die Beta-Casein-Varianten vom Typ A1 ausschließlich A1-Beta-Casein umfassen.

3. Verfahren nach Anspruch 1, wobei die Beta-Casein-Varianten vom Typ A2 ausschließlich A2-Beta-Casein umfassen.

4. Verfahren nach einem der Ansprüche 1 bis 3, wobei die Konzentrationen von drei oder vier Beta-Casein-Verdauungspeptiden, die Histidin an einer Position aufweisen, die Position 67 der Beta-Casein-Aminosäuresequenz entspricht, bestimmt werden.

5. Verfahren nach Anspruch 4, wobei die Milch Rindermilch ist und die Beta-Casein-Verdauungspeptide aus der Gruppe ausgewählt werden, umfassend:

(i) VYPFPGPIHN;
(ii) SLVYPFPGPIHN;
(iii) VYPFPGPIHNSLPQ; und
(iv) SLVYPFPGPIHNSLPQ.

6. Verfahren nach einem der Ansprüche 1 bis 3, wobei die Konzentrationen von drei oder vier Beta-Casein-Verdauungspeptiden, die Prolin an einer Position aufweisen, die Position 67 der Beta-Casein-Aminosäuresequenz entspricht, bestimmt werden.

7. Verfahren nach Anspruch 6, wobei die Milch Rindermilch ist und die Beta-Casein-Verdauungspeptide aus der Gruppe ausgewählt werden, umfassend:

(i) VYPFPGPIPN;
(ii) SLVYPFPGPIPN;
(iii) VYPFPGPIPNSLPQ; und
(iv) SLVYPFPGPIPNSLPQ.

8. Verfahren nach einem der Ansprüche 1 bis 7, wobei die unter Verwendung von Flüssigkeitschromatographie-Massenspektrometrie analysierte Mischung isotopenmarkierte Peptide enthält, die jedem der Peptide aus a) und b) von Schritt (iii) entsprechen.

9. Verfahren nach Anspruch 8, wobei die isotopenmarkierten Peptide den Verdauungspeptiden wie folgt entsprechen:

| Verdauungspeptid | Isotopenmarkiertes Peptid |
|---|---|
| VYPFPGPIHN | (*V)YPFPGPIHN |
| SLVYPFPGPIHN | SL(*V)YPFPGPIHN |
| VYPFPGPIHNSLPQ | VYPFPGPIHNS(*L)PQ |

**10.** Verfahren nach Anspruch 8, wobei die isotopenmarkierten Peptide den Verdauungspeptiden wie folgt entsprechen:

| Verdauungspeptid | Isotopenmarkiertes Peptid |
| --- | --- |
| VYPFPGPIPN | (*V)YPFPGPIPN |
| SLVYPFPGPIPN | SL(*V)YPFPGPIPN |
| VYPFPGPIPNSLPQ | VYPFPGPIPNS(*L)PQ |

**11.** Verfahren nach Anspruch 1, wobei die Milch Rindermilch ist und in Schritt (iii) die Konzentrationen der folgenden Peptide bestimmt werden:

(i) VYPFPGPIHN;
(ii) SLVYPFPGPIHN;
(iii) VYPFPGPIHNSLPQ;
(iv) SLVYPFPGPIHNSLPQ;
(v) VYPFPGPIPN;
(vi) SLVYPFPGPIPN;
(vii) VYPFPGPIPNSLPQ; und
(viii) SLVYPFPGPIPNSLPQ.

**12.** Verfahren nach einem der Ansprüche 1 bis 11, wobei die Mengen an Beta-Casein-Varianten vom Typ A1 und Beta-Casein-Varianten vom Typ A2 in der Zusammensetzung mit einer Genauigkeit von über 90 % bestimmt werden.

**13.** Verfahren nach einem der Ansprüche 1 bis 12, wobei die Mengen an Beta-Casein-Varianten vom Typ A1 und Beta-Casein-Varianten vom Typ A2 in der Zusammensetzung mit einer Genauigkeit von über 95 % bestimmt werden.

**14.** Verfahren nach einem der Ansprüche 1 bis 13, wobei die Mengen an Beta-Casein-Varianten vom Typ A1 und Beta-Casein-Varianten vom Typ A2 in der Zusammensetzung mit einer Genauigkeit von über 99 % bestimmt werden.

**15.** Verfahren nach einem der Ansprüche 1 bis 14, wobei die Zusammensetzung Rindermilch oder ein aus Rindermilch hergestelltes Milchprodukt ist.

## Revendications

**1.** Procédé permettant de déterminer les quantités de variants de bêta-caséine de type A1 ou de variants de bêta-caséine de type A2 dans une composition avec une précision supérieure à 80 %, où les variants de bêta-caséine de type A1 sont des variants de bêta-caséine comportant de l'histidine en position 67 de la séquence d'acides aminés de bêta-caséine et les variants de bêta-caséine de type A2 sont des variants de bêta-caséine comportant de la proline en position 67 de la séquence d'acides aminés de bêta-caséine, et où la composition est du lait ou un produit préparé à partir de lait, et comprenant les étapes consistant à :

(i) mettre en contact la composition avec la chymotrypsine dans des conditions permettant la digestion des bêta-caséines de la composition par la chymotrypsine ;
(ii) obtenir une solution aqueuse de peptides de digestion de bêta-caséine ;
(iii) déterminer les concentrations de :

a) deux ou plusieurs peptides de digestion de bêta-caséine présents dans la solution, dans lequel chaque peptide comprend de 8 à 20 résidus d'acide aminé et comportant de l'histidine à une position correspondant à la position 67 de la séquence d'acide aminé de bêta-caséine ; et
b) deux ou plusieurs peptides de digestion de bêta-caséine présents dans la solution, dans lequel chaque peptide comprend de 8 à 20 résidus d'acide aminé et comportant une proline à une position correspondant à la position 67 de la séquence d'acide aminé de bêta-caséine ;

en analysant le mélange par chromatographie liquide et spectrométrie de masse ; et
(iv) calculer la concentration des variants de bêta-caséine de type A1 dans la composition ou la concentration des variants de bêta-caséine de type A2 dans la composition à l'aide des concentrations de peptides de digestion de bêta-caséine déterminées à l'étape (iii).

2. Procédé selon la revendication 1, dans lequel les variants de bêta-caséine de type A1 comprennent uniquement de la bêta-caséine A1.

3. Procédé selon la revendication 1, dans lequel les variants de bêta-caséine de type A2 comprennent uniquement de la bêta-caséine A2.

4. Procédé selon l'une quelconque des revendications 1 à 3, dans lequel les concentrations de trois ou quatre peptides de digestion de bêta-caséine comportant de l'histidine à une position correspondant à la position 67 de la séquence d'acides aminés de bêta-caséine sont déterminées.

5. Procédé selon la revendication 4, dans lequel le lait est du lait bovin et les peptides de digestion de bêta-caséine sont choisis dans le groupe comprenant :

   (i) VYPFPGPIHN ;
   (ii) SLVYPFPGPIHN ;
   (iii) VYPFPGPIHNSLPQ ; et
   (iv) SLVYPFPGPIHNSLPQ.

6. Procédé selon l'une quelconque des revendications 1 à 3, dans lequel les concentrations de trois ou quatre peptides de digestion de bêta-caséine comportant une proline à une position correspondant à la position 67 de la séquence d'acides aminés de bêta-caséine sont déterminées.

7. Procédé selon la revendication 6, dans lequel le lait est du lait bovin et les peptides de digestion de bêta-caséine sont choisis dans le groupe comprenant :

   (i) VYPFPGPIPN ;
   (ii) SLVYPFPGPIPN ;
   (iii) VYPFPGPIPNSLPQ ; et
   (iv) SLVYPFPGPIPNSLPQ.

8. Procédé selon l'une quelconque des revendications 1 à 7, dans lequel le mélange analysé à l'aide de chromatographie liquide-spectrométrie de masse contient des peptides marqués isotopiquement correspondant à chacun des peptides de a) et b) de l'étape (iii).

9. Procédé selon la revendication 8, dans lequel les peptides marqués isotopiquement correspondent aux peptides de digestion comme suit :

| Peptide de digestion | Peptide marqué isotopiquement |
| --- | --- |
| VYPFPGPIHN | (*V)YPFPGPIHN |
| SLVYPFPGPIHN | SL(*V)YPFPGPIHN |
| VYPFPGPIHNSLPQ | VYPFPGPIHNS(*L)PQ |
| SLVYPFPGPIHNSLPQ | SL(*V)YPFPGPIHNSLPQ |

10. Procédé selon la revendication 8, dans lequel les peptides marqués isotopiquement correspondent aux peptides de digestion comme suit :

| Peptide de digestion | Peptide marqué isotopiquement |
| --- | --- |
| VYPFPGPIPN | (*V)YPFPGPIPN |
| SLVYPFPGPIPN | SL(*V)YPFPGPIPN |
| VYPFPGPIPNSLPQ | VYPFPGPIPNS(*L)PQ |
| SLVYPFPGPIPNSLPQ | SL(*V)YPFPGPIPNSLPQ |

11. Procédé selon la revendication 1, dans lequel le lait est du lait bovin et les concentrations des peptides suivants sont déterminées à l'étape (iii) :

(i) VYPFPGPIHN ;
(ii) SLVYPFPGPIHN ;
(iii) VYPFPGPIHNSLPQ ;
(iv) SLVYPFPGPIHNSLPQ ;
(v) VYPFPGPIPN ;
(vi) SLVYPFPGPIPN ;
(vii) VYPFPGPIPNSLPQ ; et
(viii) SLVYPFPGPIPNSLPQ.

12. Procédé selon l'une quelconque des revendications 1 à 11, dans lequel les quantités de variants de béta-caséine de type A1 et de variants de béta-caséine de type A2 dans la composition sont déterminées avec une précision supérieure à 90 %.

13. Procédé selon l'une quelconque des revendications 1 à 12, dans lequel les quantités de variants de béta-caséine de type A1 et de variants de béta-caséine de type A2 dans la composition sont déterminées avec une précision supérieure à 95 %.

14. Procédé selon l'une quelconque des revendications 1 à 13, dans lequel les quantités de variants de béta-caséine de type A1 et de variants de béta-caséine de type A2 dans la composition sont déterminées avec une précision supérieure à 99 %.

15. Procédé selon l'une quelconque des revendications 1 à 14, dans lequel la composition est du lait ou un produit laitier préparé à partir de lait bovin.

Figure 1

## Relative Standards - AQ vs Actual

$y = 0.9861x$
$R^2 = 0.9996$

Figure 2

## Milk Products - AQ vs Calculated bCN

- ■ Milk
- ▲ Milk Powder
- ● UHT Milk
- ✕ Infant Formula

$y = 1.1016x$
$R^2 = 0.9075$

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- CN 108519485 A
- WO 1996014577 A
- WO 1996036239 A
- WO 2002019832 A
- WO 2014193248 A
- WO 2015005804 A
- WO 2015026245 A
- WO 2017171563 A
- WO 2018063008 A

**Non-patent literature cited in the description**

- **TRIVEDI, M.S.** ; **SHAH, J.S** ; **AL-MUGHAIRY, S.** ; **HODGSON, N.W.** ; **SIMMS, B.** ; **TROOSKENS, G.** ; **VAN CRIEKINGE, W.** ; **DETH, R.C.** *J. Nutr. Biochem*, 2014, vol. 25, 1011-1018
- **VINCENT, D.** ; **ELKINS, A** ; **CONDINA, M.R.** ; **EZERNIEKS, V** ; **ROCHFORT, S.** *PLOS ONE*, 2016, vol. 11, e0163471
- **GIVENS, I.** ; **AIKMAN, P.** ; **GIBSON, T.** ; **BROWN, R**. *Food Chemistry*, 2013, vol. 139, 549-552
- **LUTTER, P.** ; **PARISOD, V.** ; **WEYMUTH, H.** *J. AOAC Int.*, 2011, vol. 94, 1043-1059